# EUROPEAN PATENT SPECIFICATION

(11) **EP 0 400 676 B1**
(45) Date of publication and mention of the grant of the patent: **06.10.1993**
(21) Application number: 90110494.3
(22) Date of filing: 01.06.1990
(51) Int. Cl.: A61L 11/00

(54) **Medical waste crusher with hot-air sterilizer**
Medizinische Abfallzerkleinerungsanlage mit einem Heissluftsterilisator
Installation de broyage pour déchets médicaux contenant un stérilisateur à air chaud

(30) Priority: 02.06.1989 JP 140724/89
(43) Date of publication of application: 05.12.1990
(73) Proprietor: THE HANSHIN DIESEL WORKS, LTD., Kobe-shi, Hyogo-ken (JP)
(72) Inventor: Matsumoto, Masayuki, Kobe-shi, Hyogo-ken (JP); Mori, Hikozumi, Kobe-shi, Hyogo-ken (JP)
(74) Representative: Patentanwälte Leinweber & Zimmermann

(56) References cited:
- DE-A- 3 400 189
- DE-C- 3 604 582

## Description

The present invention relates to a sterilizing crusher used for the safe disposal of medical waste such as used medical treatment aids including infusion units, syringes, etc.

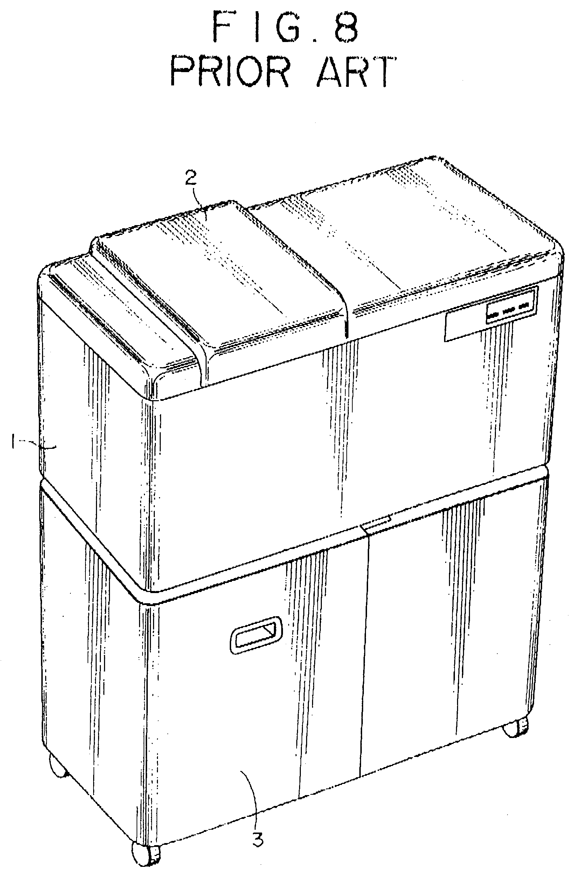

Sterilizing crushers of the type described are known, which include, as shown in Figure 8 of the accompanying drawings, a processing chamber 1 in which used medical treatment aids such as infusion units, syringes, etc. are sterilized and crushed. The processing chamber 1 has an inlet normally closed by a top cover or lid 2 and an outlet (not shown) from which sterilized and crushed medical treatment aids are discharged into a refuse bag (not shown) set in a dust box 3. The refuse bag filled with the sterilized and crushed medical treatment aids is removed from the dust box 3 and is thrown away in a known manner.

In the processing chamber 1, the used medical treatment aids are crushed while being sterilized with a strong medical fluid sprayed into the processing chamber 1. Since the medical fluid is per se detrimental to human beings, the medical treatment aids sterilized with such detrimental medical fluid constitute a secondary contaminant. Furthermore, spraying is insufficient to provide a uniform distribution of the medical fluid over the entire region of the processing chamber 1, with the result that a necessary sterilizing effect is difficult to obtain.

With the foregoing drawbacks of the prior art in view, it is an object of the present invention to provide a sterilizing crusher incorporating structural features which enable a safe disposal of medical waste without involving a secondary contamination.

In brief, a sterilizing crusher of the present invention includes a hot-air sterilizer disposed upstream of a crushing unit. The hot-air sterilizer is kept at a temperature in the range from 160°C to 200°C so that the used medical treatment aids are completely sterilized before they are crushed into fine dust by means of the crushing unit. Since the sterilization is accomplished by hot air instead of a medical fluid, the secondary contamination is never caused by the crushed fine dust.

More particularly, according to the present invention, there is provided an apparatus for sterilizing and crushing contaminated materials made of light metal, paper, glass, synthetic resin, rubber, etc., the apparatus comprising: a heat sterilization chamber having a top lid openable for receiving the contaminated materials in the heat sterilization chamber, a bottom lid openable for discharging the materials from the heat sterilization chamber after the materials are sterilized, and an air intake hole and an air discharge hole that are formed in an upper half of the heat sterilization chamber in confrontation to one another, the heat sterilization chamber having a heat-resistant non-sticking coating on its entire inside surface; means for circulating hot air through the heat sterilization chamber via the air intake and discharge holes so as to heat the sterilization chamber at a temperature in the range from 160°C to 200°C for a predetermined period of time; and a power-driven crushing unit disposed below the heat sterilization chamber for crushing the sterilized material discharged from the heat sterilization chamber.

The above and other objects, features and advantages of the present invention will become more apparent from the following description when making reference to the detailed description and the accompanying sheets of drawings in which a preferred structural embodiment incorporating the principles of the present invention is shown by way of illustrative example.
Figure 1 is a front elevational view of a medical waste crushing apparatus including a hot-air sterilizer according to the present invention;
Figure 2 is a right side view of Figure 1;
Figure 3 is a plan view of Figure 1;
Figure 4 is a cross-sectional view taken along line IV - IV of Figure 2;
Figure 5 is a diagrammatical exploded perspective view illustrative of the operation of the apparatus;
Figure 6 is a vertical cross-sectional view showing a heat sterilization chamber and related parts of the apparatus;
Figure 7 is a cross-sectional view taken along line VII - VII of Figure 6; and
Figure 8 is a perspective view of a conventional sterilizing crusher.

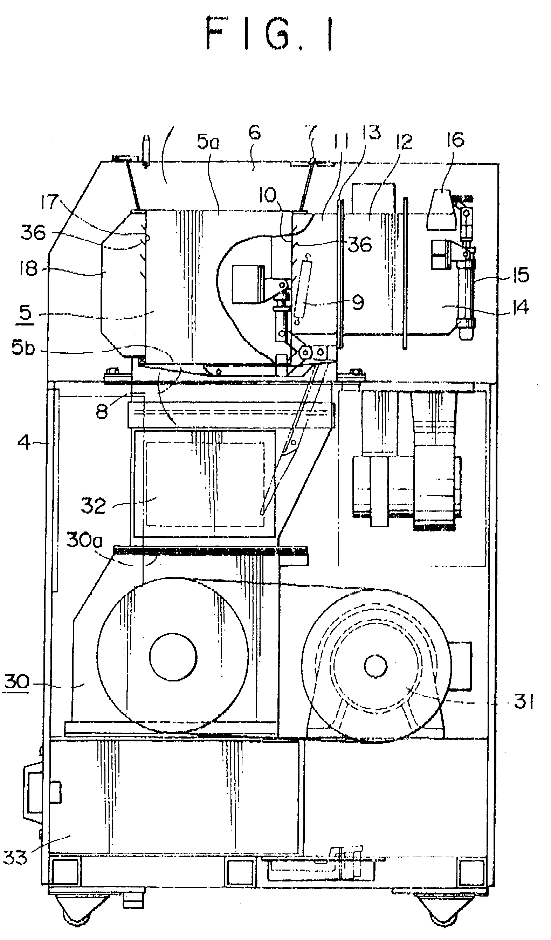

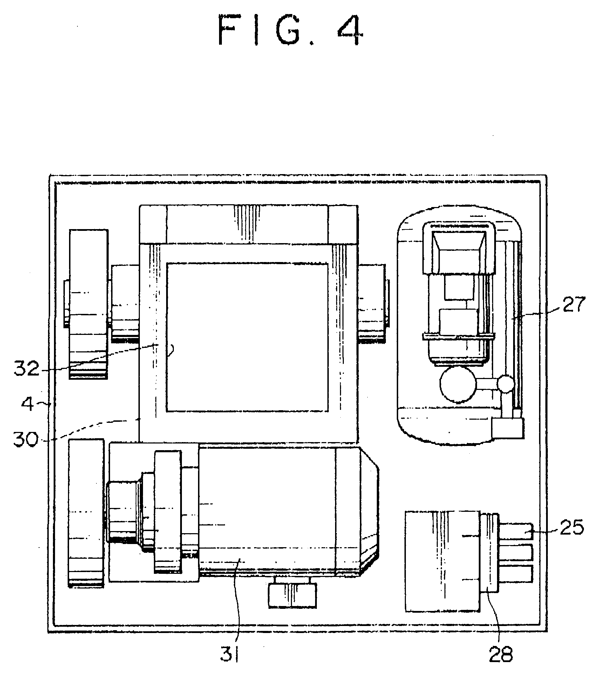

The present invention will be described below in greater detail with reference to an embodiment shown in Figures 1 through 7 of the accompanying drawings.

As shown in Figure 1, a sterilizing crusher embodying the present invention generally comprises a hot air sterilizer mounted in an upper part of a hollow rectangular body or frame 4, and a power-driven crushing unit disposed in the lower part of the frame 4.

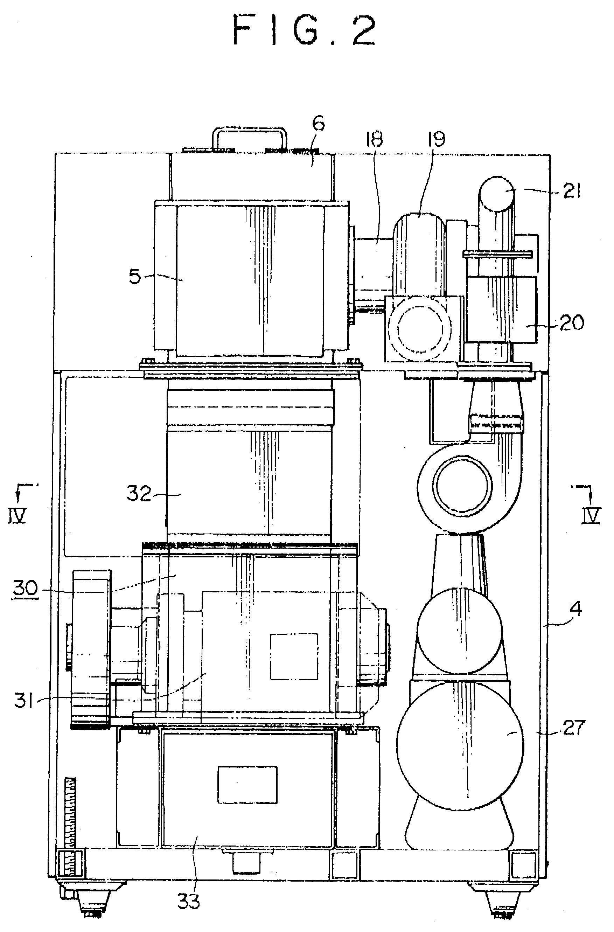

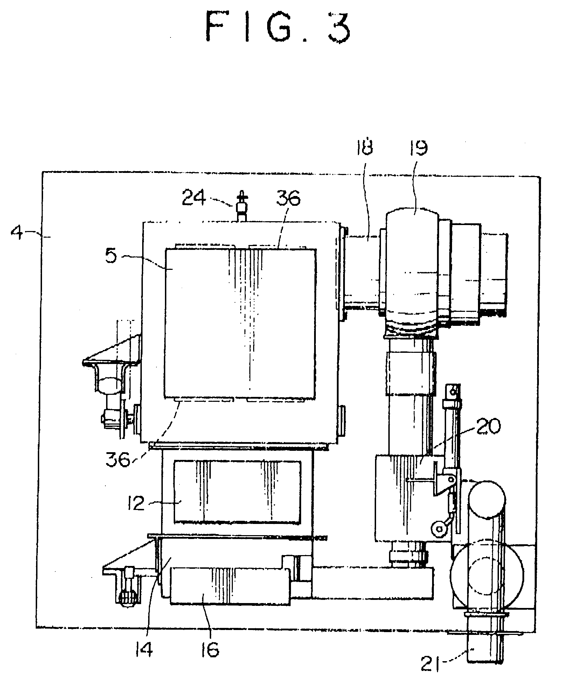

The hot air sterilizer includes a heat sterilization chamber 5 and means for circulating hot air through the heat sterilization chamber 5 to sterilize medical waste such as used treatment aids including infusion units, syringes, etc. received in the heat sterilization chamber 5. The heat sterilization chamber 5 has a substantially rectangular box-like shape having an inlet 5a and an outlet 5b defined in top and bottom walls, respectively, of the box-like heat sterilization chamber 5. The inlet 5a is normally closed by a top cover or lid 6 attached to the frame 4 by a pair of hinges 7 (only one shown). The outlet 5b is normally closed by a bottom cover or lid 8 which is driven by a damper mechanism 9 for opening and closing the outlet 5b. The box-like heat sterilization chamber 5 further includes a plurality of air intake holes 10 formed in an upper half of one side wall of the heat sterilization chamber 5 for introducing hot air into the heat sterilization chamber 5. The air intake holes 10 are held in fluid communication with a supply duct 11 connected at its one end to the side wall of the heat sterilization chamber 5. The opposite end of the supply duct 11 is connected to a heater 12, with an air distribution plate 13 having a plurality of small holes or perforations 13a disposed between the supply duct 11 and the heater 12. With the perforated air distribution plate 13 thus provided, air heated by the heater 12 is supplied uniformly over the entire area of the supplied duct 11. The heater 12 is connected to a gas distribution box 14 having an air intake damper 16 adapted to be opened and closed by an air cylinder 15 for introducing outside air into the heat sterilization chamber 5. A plurality of air discharge holes 17 are formed in an upper half of one side wall of the box-like heat sterilization chamber 5 in confronting relation to the air intake holes 10 for permitting hot air to evade from the heat sterilization chamber 5. The air discharge holes 17 are kept in fluid communication with a return duct 18 connected at its one end to another side wall of the heat sterilization chamber 5. The other end of the return duct 18 is connected to a blower 19 (Figures 2 and 3) for forcing air to circulate through the heat sterilization chamber 5.

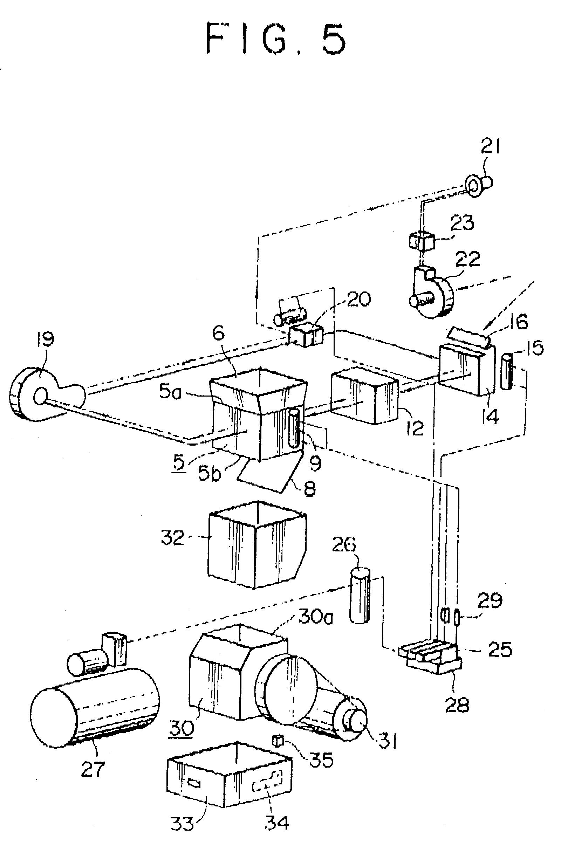

The blower 19 is connected to a selector damper 20 which in turn is connected to an exhaust pipe 21 and the gas distribution box 14, as shown in Figure 3. Thus, the air flow path extending from the blower 19 is branched by the selector damper 20 into two sides, namely the side of the exhaust pipe 21 and the side of the gas distribution box 14. The selector damper 20 is operative to switch or changeover the air flow path between the exhaust pipe side and the gas distribution box side. As shown in Figure 5, an exhaust fan 22 is connected to the exhaust pipe 21 via a check valve 23. A solenoid-operated value unit 25 having three solenoid-operated valves arranged in tandem relation is connected with the damper mechanism 9, the air cylinder 15 and the selector damper 20 for controlling the operation of them. The solenoid-operated valve unit 25 is also connected through an air regulator 26 to an air compressor 27. The respective valves of the solenoid-operated valve unit 25 are connected together by a manifold 28. A speed controller 29 is disposed between the damper mechanism 9 and the solenoid-operated valve 25 for controlling the speed of the opening and closing movement of the bottom lid 8.

Referring back to Figure 1, the power-driven crushing unit includes a box-like crushing chamber 30 and is mounted in a lower portion of the frame 4. The crushing unit further includes an electric motor 31 for driving a rotor with three crushing edges (not shown) disposed in the crushing chamber 30 for crushing the sterilized medical waste. A hopper 32 is disposed between the outlet 5b of the heat sterilization chamber 5 and an inlet 30a of the crushing chamber 30 for receiving the sterilized medical waste discharged from the heat sterilization chamber 5 and supplying the thus received medical waste into the crushing chamber 30. A removable dust box 33 is disposed below the crushing chamber 30 for collecting therein crushed medical waste. The dust box 33 is provided with a weighing device 34 as shown in Figure 5, so that when a predetermined amount of crushed medical waste is collected in the dust box 33, the dust box 33 is removed from the frame 4 for the subsequent disposal of the sterilized crushed medical waste. Designated by 35 is a microswitch for detecting the presence of the dust box 33 so as to avoid activation of the crusher in the absence of the dust box 33.

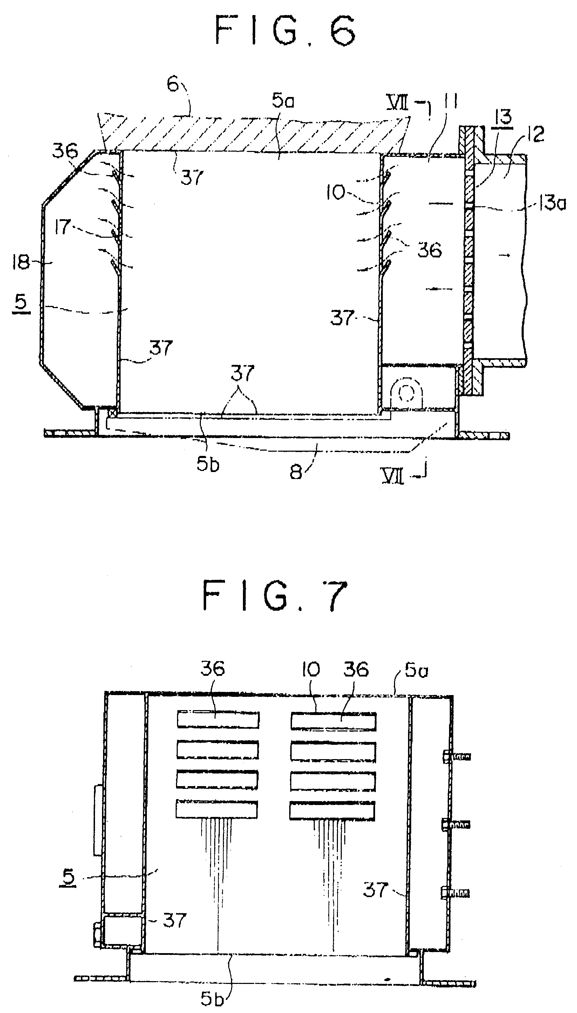

As best shown in Figures 6 and 7, the air intake holes 10 and the air discharge holes 17 are substantially uniformly distributed over an upper half of the heat sterilization chamber 5. Each of the air intake and discharge holes 10, 17 has a rectangular shape and is furnished with one slat or fin 36 sloping upwardly and outwardly from a lower edge of each hole 10, 17. The entire inside surface of the heat sterilization chamber 30 including the under surface of the top lid 6 and the upper surface of the bottom lid 8 has a heat-resistant, non-adherent or non-sticking coating 37 such as a Teflon coating ("Teflon" is the trademark for polytetrafluoroethylene) for a purpose described below.

The sterilizing crusher of the foregoing construction operates as follows.

The top lid 6 is pulled to open the inlet 5a and then medical waste such as used treatment aids is charged from the inlet 5a into the heat sterilization chamber 5. After the top lid 6 is closed, a start switch (not shown) is turned on, whereupon operation of the heater 12, the blower 19, the air compressor 27 and the solenoid-operated valve unit 25 is started. In this instance, the air intake damper 16 is closed and the selector damper 20 is set in a position to connect the blower 19 and the gas distribution box 14. Thus, there is completed a closed air flow path extending through the blower 19, the selector damper 20, the gas distribution box 14, the heater 12 and the heat sterilization chamber 5, as indicated by solid lines shown in Figure 5. Air as it flows through the heater 12 is heated at a temperature of 180°C. Then, hot air is uniformly distributed into the heat sterilization chamber 5 by the perforated air distribution plate 13. The heat sterilization chamber 5 is heated at 180°C for about 30 minutes. During that time, the used medical treatment aids are completely sterilized in the heat sterilization chamber 5.

Then, the heater 12 is de-energized, the solenoid-operated valve unit 25 is activated to place the selector damper 20 in a position to connect the blower 19 and the exhaust pipe 21. The solenoid-operated valve unit 25 also actuates the air cylinder 15 to open the air intake damper 16. Thus, the outside air is drawn from the air intake damper 16 into the gas distribution box 14 and then flows successively through the heater 12, the heat sterilization chamber 5, the blower 19, and the selector damper 20 toward the exhaust pipe 21, as indicated by broken lines shown in Figure 5. Thus, hot air is expelled from the heat sterilization chamber 5 to the atmosphere and the heat sterilization chamber 5 is cooled to a temperature of 70°C.

The blower 19 is de-energized and the motor 31 of the crushing unit is driven to rotate the crusher motor disposed inside the crushing chamber 30. Then, the solenoid-operated valve unit 25 is driven to actuate the damper mechanism 8 in a direction to open the bottom lid 8 whereupon the sterilized medical waste is discharged from the outlet 5b into the hopper 32 which in turn supplied the sterilized medical waste into the crushing chamber 30. The sterilized medical waste thus supplied is crushed by the rotating crushing rotor and then is collected into the dust box 33. After the medical waste supplied from the hopper 32 is completely crushed, a stop switch (not shown) is turned on to stop operation of the sterilizing crusher. The medical waste collected in the dust box 30 is crushed into fine dust and free from germ, so that a subsequent disposal of the thus treated medical waste is achieved safely with utmost ease.

In the embodiment described above, the sterilization is achieved by heating the heat sterilization chamber 5 at 180°C for 30 minutes, however, the same sterilizing effect can be obtained by sterilization at 160°C for 60 minutes. For the effective sterilization, the necessary heating temperature is at least 160°C. The heating at a temperature exceeding 200°C is not preferable because an offensive smell is sent out from the medical waste being sterilized by heat. It is therefore necessary to maintain the sterilization temperature within a range from 160°C to 200°C. Within this temperature range, various temperatures may be used to accomplish the desired sterilization in which instance the heating time period is set to vary in inverse proportion to the sterilization temperature.

As described above, the air flow path formed during the heat sterilization is closed as indicated by the solid lines of Figure 1, and hence the heat sterilization can be accomplished at safety without the occurrence of leakage of an offensive smell and a noxious gas to the outside of the sterilizing crusher even when the medical waste is heated at 200°C.

The medical waste made of plastic is melted down when heated at a temperature ranging from 160°C to 200°C. However, overflow of such molten plastic material does not take place because the air intake holes 10 and the air discharge holes 17 are formed in an upper half of the heat sterilization chamber 5 and because the upwardly sloping fins 36 are provided for the respective air intake and discharge holes 10, 17. Since the inside surface of the heat sterilization chamber 5 is entirely coated with the heat-resistant non-sticking material such as Teflon, the molten medical waste flows smoothly from the heat sterilization chamber 5 to the crushing chamber 30 when the bottom lid 5b is opened. The heat sterilization chamber 5 having such heat-resistant non-sticking coating 37 is easy to maintain and has a long service life. Other heat-resistant non-sticking coating materials may be used in place of the Teflon coating.

### Example

The sterilizing crusher of the embodiment described above was tested for sterilizing performance by using blood-collecting tubes contaminated with particular bacteria described below.

Five groups of blood-collecting tubes contaminated with Staphylococcus aureus, Escherichia coli, Pseudomonas aeruginosa, Bacillus substilis, and Candida albicans, respectively were prepared. Each group of the contaminated blood-collecting tubes were sterilized in the heat sterilization chamber of the sterilizing crusher. The blood-collecting tubes recovered from the heat sterilization chamber were tested for the presence of the bacteria. The test results indicated that as to four groups of the blood-collecting tubes contaminated with vegetative bacteria, namely Staphylococcus aureus, Escherichia coli, Pseudomonas aeruginosa, and Candida albicans, a germ-free condition was obtained by sterilization at 120°C for 15 minutes, while as to the remaining group contaminated with Bacillus substilis which is sporogenic and highly resistive to heat, a germ-free condition was obtained by sterilization at 180°C for 30 minutes. The same sterilization effect against Bacillus substilis was obtained by heating at 160°C for 60 minutes.

As described above, the medical waste is sterilized by hot air without using a detrimental medical fluid with the result that a safe disposal of the medical waste can be achieved.

## Claims

1. An apparatus for sterilizing and crushing contaminated materials made of light metal, paper, glass, synthetic resin, rubber, etc., said apparatus comprising:
(a) a heat sterilization chamber (5) having a top lid (16) openable for receiving the contaminated materials in said heat sterilization chamber (5), a bottom lid (8) openable for discharging the materials from said heat sterilization chamber (5) after the materials are sterilized, and an air intake hole (10) and an air discharge hole (17) that are formed in an upper half of said heat sterilization chamber (5) in confrontation to one another, said heat sterilization chamber (5) having a heat-resistant non-sticking coating (37) on its entire inside surface;
(b) means for circulating hot air through said heat sterilization chamber (5) via said air intake and discharge holes (10, 17) so as to heat said sterilization chamber (5) at a temperature in the range from 160°C to 200°C for a predetermined period of time; and
(c) a power-driven crushing unit (30, 31) disposed below said heat sterilization chamber (5) for crushing the sterilized material discharged from said heat sterilization chamber (5).

2. An apparatus according to claim 1 wherein said heat sterilization chamber (5) further includes a fin (36) sloping upwardly and outwardly from a lower edge of each of said air intake and discharge holes (10, 17).

3. An apparatus according to anyone of claims 1 or 2 wherein said heat-resistant non-sticking coating (37) is a polytetrafluoroethylene coating.

4. An apparatus according to anyone of claims 1 to 3 wherein said circulating means includes a heater (12) disposed on one side of said heat sterilization chamber (5) adjacent to said air intake hole (10), and a blower (19) disposed on the opposite side of said heat sterilization chamber (5) adjacent to said air discharge hole (17) for forcing air through said heater (12) toward said air intake hole (10).

5. An apparatus according to claim 4 wherein said circulating means further includes a perforated air distribution plate (13) disposed between said heat sterilization chamber (5) and said heater (12).

6. An apparatus according to anyone of claims 4 or 5 wherein said circulating means further includes a gas distribution box (14) disposed between said heater (12) and said blower (19) for introducing the outside air into said heat sterilization chamber (5), an exhaust pipe (21) for permitting hot air to evade from said heat sterilization chamber (5), and a selector damper (20) for selectively connecting said blower (19) and said gas distribution box (14) and said blower (19) and said exhaust pipe (21).

7. An apparatus according to claim 6 wherein said circulating means further includes an exhaust fan (22) associated with said exhaust pipe (21).

8. An apparatus according to anyone of claims 1 to 7, further including a damper mechanism (9) for opening and closing said bottom lid (6).

9. An apparatus according to anyone of claims 1 to 8 wherein said power-driven crushing unit includes a crushing chamber (30) disposed below said heat sterilization chamber (5), a hopper (32) disposed between said bottom lid (8) and said crushing chamber (30), and a removable dust box (33) disposed below said crushing chamber (30) for collecting the sterilized and crushed materials.

## Patentansprüche

1. Vorrichtung zum Sterilisieren und Zerkleinern von kontaminierten Materialien aus Leichtmetall, Papier, Glas, Kunststoff, Gummi usw., umfassend:
(a) eine Hitzesterilisationskammer (5) mit einem oberen Deckel (6), der zum Einbringen der kontaminierten Materialien in die Hitzesterilisationskammer (5) geöffnet werden kann, mit einem unteren Deckel (8), der zum Abführen der Materialien aus der Hitzesterilisationskammer (5) nach deren Sterilisation geöffnet werden kann, und mit einer Lufteinlaßöffnung (10) und einer Luftauslaßöffnung (17), die in einer oberen Hälfte der Hitzesterilisationskammer (5) in einander gegenüberliegender Beziehung angeordnet sind, wobei die Hitzesterilisationskammer (5) an ihrer gesamten Innenfläche eine hitzefeste, nicht-klebrige Beschichtung (37) aufweist;
(b) Mittel zum Umwälzen heißer Luft durch die Hitzesterilisationskammer (5) über die Lufteinlaß- und auslaßöffnung (10, 17), um die Sterilisationskammer (5) für eine bestimmte Zeitdauer auf eine Temperatur im Bereich vom 160 bis 200°C zu erhitzen; und
(c) eine angetriebene Zerkleinerungseinheit (30, 31), die unterhalb der Sterilisationskammer (5) angeordnet ist, um das aus der Hitzesterilisationskammer (5) ausgetragene sterilisierte Material zu zerkleinern.

2. Vorrichtung nach Anspruch 1, wobei die Hitzesterilisationskammer (5) ferner ein Leitblech aufweist, das von einer Unterkante jeder der besagten Lufteinlaß- und Luftauslaßöffnungen (10, 17) nach oben und nach außen geneigt ist.

3. Vorrichtung nach Anspruch 1 oder 2, wobei die hitzefeste, nicht-klebrige Beschichtung (37) eine Beschichtung aus Polytetrafluorethylen ist.

4. Vorrichtung nach einem der Ansprüche 1 bis 3, wobei die Umwälzmittel eine Heizeinrichtung (12), die an einer Seite der Hitzesterilisationskammer (5) neben der Lufteinlaßöffnung (10) angeordnet ist, und ein Gebläse (19) umfassen, die an der gegenüberliegenden Seite der Hitzesterilisationskammer (5) neben der Luftauslaßöffnung (17) angeordnet ist, um Luft durch die Heizeinrichtung (12) hindurch der Lufteinlaßöffnung (10) zuzuführen.

5. Vorrichtung nach Anspruch 4, wobei die Umwälzmittel ferner eine perforierte Luftverteilerplatte (13) umfassen, die zwischen der Hitzesterilisationskammer (5) und der Heizeinrichtung (12) angeordnet ist.

6. Vorrichtung nach einem der Ansprüche 4 oder 5, wobei die Umwälzmittel ferner eine Gasverteilerkammer (14), die zwischen der Heizeinrichtung (12) und dem Geblläse (19) angeordnet ist, um Außenluft in die Hitzesterilisationskammer (5) einzuführen, ein Ausstoßrohr (21) zum Ableiten von heißer Luft aus der Hitzesterilisationskammer (5) und eine Auswahldrosselklappe (20) umfassen, um wahlweise das Gebläse (19) und die Gasverteilerkammer (14) oder das Gebläse (19) und das Ausstoßrohr (21) miteinander zu verbinden.

7. Vorrichtung nach Anspruch 6, wobei die Umwälzmittel ferner ein dem Ausstoßrohr (21) zugeordnetes Ausstoßgebläse (22) umfassen.

8. Vorrichtung nach einem der Ansprüche 1 bis 7 ferner umfassend einen Dämpfermechanismus (9) zum Öffnen und Schließen des unteren Deckels (6).

9. Vorrichtung nach einem der Ansprüche 1 bis 8, wobei die angetriebene Zerkleinerungseinheit ferner eine unter der Sterilisationskammer (5) angeordnete Zerkleinerungskammer (30), einen zwischen dem unteren Deckel (8) und der Zerkleinerungskammer (30) angeordneten Einfüllschacht (32) und einen Staubbehälter (33) umfaßt, der unter der Zerkleinerungskammer (30) angeordnet ist, um die zerkleinerten und sterilisierten Materialien zu sammeln.

## Revendications

1. Appareil pour stériliser et broyer des matériaux contaminés en métal léger, en papier, en verre, en résine synthétique, en caoutchouc, etc., ledit appareil comprenant :
(a) une chambre (5) de stérilisation à chaud comportant un couvercle supérieur (6) pouvant être ouvert pour la réception des matériaux contaminés dans ladite chambre (5) de stérilisation à chaud, un couvercle inférieur (8) pouvant être ouvert pour décharger les matériaux provenant de ladite chambre (5) de stérilisation à chaud après que les matériaux ont été stérilisés, et un trou (10) d'admission d'air ainsi qu'un trou (17) d'évacuation d'air qui sont formés dans la moitié supérieure de ladite chambre (5) de stérilisation à chaud, en regard l'un de l'autre, ladite chambre (5) de stérilisation à chaud comportant sur toute sa surface intérieure un revêtement (37) non-collant et résistant à la chaleur ;
(b) un moyen pour faire circuler l'air chaud à travers ladite chambre (5) de stérilisation à chaud par l'intermédiaire desdits trous (10, 17) d'admission et d'évacuation de manière à chauffer la chambre (5) de stérilisation à chaud à une température comprise entre 160°C et 200°C pendant une période de temps prédéterminée ; et
(c) un dispositif de broyage (30, 31) entraîné mécaniquement et disposé en dessous de la chambre (5) de stérilisation à chaud pour broyer le matériau stérilisé évacué de la chambre (5) de stérilisation à chaud.

2. Appareil selon la revendication 1, dans lequel la chambre (5) de stérilisation à chaud comprend, en outre, une ailette (36) s'inclinant vers le haut et vers l'extérieur depuis le bord inférieur de chacun des trous d'admission et d'évacuation (10, 17).

3. Appareil selon l'une quelconque des revendications 1 ou 2, dans lequel le revêtement (37) non-collant et résistant à la chaleur est un revêtement de polytétrafluoroéthylène.

4. Appareil selon l'une quelconque des revendications 1 à 3, dans lequel le moyen de circulation comprend un dispositif de chauffage (12) disposé sur un des côtés de la chambre (5) de stérilisation à chaud au voisinage dudit trou (10) d'admission d'air, et une soufflante (19) disposée sur le côté opposé de la chambre (5) de stérilisation à chaud au voisinage dudit trou (17) d'évacuation d'air pour refouler l'air à travers le dispositif de chauffage (12) en direction du trou (10) d'admission d'air.

5. Appareil selon la revendication 4, dans lequel ledit moyen de circulation comprend, en outre, une plaque perforée (13) de distribution d'air disposée entre la chambre (5) de stérilisation à chaud et le dispositif de chauffage (12).

6. Appareil selon l'une quelconque des revendications 4 ou 5, dans lequel le moyen de circulation comprend, en outre, une boîte (14) de distribution de gaz disposée entre le dispositif de chauffage (12) et la soufflante (19) pour introduire l'air extérieur dans la chambre (5) de stérilisation à chaud, un tuyau d'échappement (21) pour permettre à l'air chaud de s'échapper de la chambre (5) de stérilisation à chaud, et un registre sélecteur (20) pour raccorder sélectivement la soufflante (19) et la boîte (14) de distribution de gaz ainsi que la soufflante (19) et le tuyau d'échappement (21).

7. Appareil selon la revendication 6, dans lequel le moyen de circulation comprend, en outre, un ventilateur d'échappement (22) associé au tuyau d'échappement (21).

8. Appareil selon l'une quelconque des revendications 1 à 7, comprenant, en outre, un mécanisme (9) d'actionnement de registre pour ouvrir et fermer le couvercle inférieur (6).

9. Appareil selon l'une quelconque des revendications 1 à 8, dans lequel le dispositif de broyage entraîné mécaniquement comprend une chambre de broyage (30) disposée en dessous de la chambre (5) de stérilisation à chaud, une trémie (32) disposée entre le couvercle inférieur (8) et la chambre de broyage (30), et une boîte à poussière amovible (33) disposée en dessous de la chambre de broyage (30) pour recueillir les matériaux stérilisés et broyés.
